# EUROPÄISCHE PATENTSCHRIFT

(11) **EP 1 658 876 B1**
(45) Veröffentlichungstag und Bekanntmachung des Hinweises auf die Patenterteilung: **21.02.2007**
(21) Anmeldenummer: 05009940.7
(22) Anmeldetag: 06.05.2005
(51) Int. Cl.: A61M 25/06, A61M 5/158

(54) **Auszieheinrichtung zum geschützten Herausziehen einer flexiblen Punktionsnadel aus einem Katheter**
Withdrawal apparatus used for protected withdrawing a flexible puncture needle from a catheter
Dispositif d'extraction pour extraire de façon protégée une aiguille flexible à ponction d'un cathéter

(30) Priorität: 17.11.2004 DE 202004017861 U
(43) Veröffentlichungstag der Anmeldung: 24.05.2006
(73) Patentinhaber: Clinico GmbH, 36251 Bad Hersfeld (DE)
(72) Erfinder: Heinzerling, Jörg, 36251 Bad Hersfeld (DE); Pütter, Harry, 36364 Bad Salzschlirf (DE)
(74) Vertreter: von Eichel-Streiber, Caspar

(56) Entgegenhaltungen:
- EP-A- 1 331 018
- WO-A-02/100263
- US-A- 5 476 106
- US-A- 6 056 718
- US-A1- 2004 158 207

## Beschreibung

Die Erfindung betrifft eine Auszieheinrichtung zum geschützten Herausziehen einer Punktionsnadel aus einem Katheter, insbesondere einem Katheter mit flexiblem Verweilschlauch.

In der Medizin werden in verschiedenen Bereichen zur Verabreichung von in Flüssigkeit gelösten oder suspendierten Medikamenten Katheter verwendet, durch die über eine Punktionsstelle die Medikation direkt in das Gewebe oder eine Blutbahn des Patienten verabreicht wird. Dabei muss zum Anlegen des Katheters zunächst ein Zugang zum Gewebe bzw. der Blutbahn des Patienten durch Punktion geschaffen werden. Hierfür sind verschiedentliche Punktionsanordnungen bekannt , siche z. B. die Patentschriften WO-A-02/100 263 und EP-A-1 331 018.

Die Patentschrift WO-A-02/100263 offenbart eine Auszieheinrichtung zum geschützten Herausziehen einer Punktionsnadel aus einem Katheter mittels einer an der Punktionsnadel befestigten flexiblen Schnur, wobei die flexible Schnur um einen Bogen gezogen wird.

Die Patentschrift EP-A-1 331 018 offenbart eine Einrichtung zum geschützten Herausziehen einer Nadel mittels eines Zahnrades und einer Zahnstangenleiste.

Punktionsanordnungen dieser Art sind häufig auch für die eigenständige Anwendung durch den Patienten selbst, beispielsweise für die Insulinverabreichung mit Hilfe einer Insulinpumpe, gedacht.

Bei Punktionsanordnungen mit flexiblem Verweilschlauch und einer Punktionsnadel wird zugleich mit dem Aufsetzen des Katheteranschlussstückes die Hautoberfläche und das darunter liegende Gewebe des Patienten durch die Punktionsnadel punktiert und in diesem Zuge der flexible Verweilschlauch in das Gewebe eingebracht. Als Einstichpunkt für die Punktionsanordnung wird dabei häufig der Bauchbereich des Patienten gewählt. Nach dem Einstechen muss die Punktionsnadel zurückgezogen und entfernt werden, und es verbleibt in der Punktionsstelle lediglich der Verweilschlauch. Anschließend wird an das Katheteranschlussstück eine entsprechende Versorgungsleitung und somit eine Medikamentenversorgung angeschlossen. Das Zurückziehen der Punktionsnadel soll zum einen schnell und sicher erfolgen, zum anderen soll die Punktionsnadel dabei nach Möglichkeit nach dem Zurückziehen gekapselt, zumindest aber gegen offenen Zugang geschützt sein, um die Gefahr von Verletzungen mit der mit Körperflüssigkeiten kontaminierten Nadel und damit auch die Gefahr von Infektionen zu verringern, möglichst vollständig auszuschlie-ßen.

Diesen Ansprüchen soll eine erfindungsgemäße Auszieheinrichtung genügen.

Hierzu wird erfindungsgemäß eine Auszieheinrichtung zum geschützten Herausziehen einer flexiblen Punktionsnadel aus einem Katheter, insbesondere einem Katheter mit flexiblem Verweilschlauch, nach dem Punktieren vorgeschlagen, mit den Merkmalen des Schutzanspruchs 1. Vorteilhafte Weiterbildungen der erfindungsgemäßen Auszieheinrichtung sind in den abhängigen Schutzansprüchen 2 bis 9 angegeben.

Der eigentliche _{"}Witz" der erfindungsgemäßen Auszieheinrichtung liegt darin begründet, dass zum Herausziehen der Punktionsnadel aus dem Katheter ein einfaches "Getriebe" verwendet wird. Das rückwärtige Ende der Punktionsnadel ist an dem Zahnrad so festgelegt, dass es beim Zurückziehen des Ruckzughebels und die dabei durch Zusammenwirken der Umfangsverzahnung des Zahnrades mit der linearen Verzahnung entstehende Rotation des Zahnrades auf den Aufwickelabschnitt des Zahnrades aufgewickelt wird. Die Punktionsnadel wird so mit ihrem spitzen Ende aus dem Katheter heraus und in die Auszieheinrichtung hinein gezogen.

In einem Gebrauchsanfangszustand wird die das Führungsteil und den Rückzughebel aufweisende Auszieheinrichtung an ihrem Verbindungsabschnitt mit einem entsprechend komplementär gestalteten Verbindungsabschnitt des Katheters verbunden. In diesem Gebrauchsanfangszustand wird die aus der Auszieheinrichtung und dem Katheter bestehender Einheit ausgeliefert, so dass ein Anwender den Katheter setzen kann. Nach dem Setzen des Katheters zieht der Anwender durch Verschieben des Rückzughebels die Punktionsnadel aus dem Katheter heraus und löst die Auszieheinrichtung von dem Katheter. Dann ist das Katheter bereit zum Anschließen einer Versorgungsleitung.

Vorzugsweise ist die lineare Verzahnung an dem Führungsteil angeordnet und das Zahnrad auf einem mit dem Rückzughebel fest verbundenen Zapfen drehbar gelagert. Diese Anordnung hat den Vorteil, dass bei einem Zurückziehen des Rückzughebels nicht allein das Aufwickeln des rückwärtigen Endes der Punktionsnadel ein Herausziehen der Nadel aus dem Katheter bewirkt, sondern auch die lineare Bewegung des Zahnrades mit dem Rückzughebel. Auf diese Weise wird ein doppelter Auszieheffekt erzeugt, wodurch die Auszieheinrichtung insgesamt kompakter gebaut werden kann.

Um die für das Herausziehen der Punktionsnadel aus dem Katheter erforderliche Kraft zu erzeugen, ohne eine übermäßige Kraft beim Zurückziehen des Rückzughebels zu verlangen, oder um den erforderlichen Weg des Rückzughebels möglichst kurz zu halten, ist es sinnvoll, die lineare Verzahnung und die Umfangsverzahnung so zu dimensionieren, dass sich eine für die genannten Ziele sinnvolle Über- bzw. Untersetzung ergibt.

Eine gemäß den Merkmalen des Anspruchs 4 in vorteilhafter Weise weitergebildete Auszieheinrichtung bietet einen sicheren Schutz für die Spitze der Punktionsnadel nach deren Herausziehen, so dass es nicht zu versehentlichen Verletzungen aufgrund unsachgemäßen Behandelns der Punktionsnadel und einer damit verbundenen Infektionsgefahr kommen kann.

Um ein Verschieben (in der Regel Zurückziehen) des Rückzughebels zu vereinfachen, weist dieser in einer vorteilhaften Weiterbildung einen Griffabschnitt auf. Als Gegenlager kann auch an dem Führungsteil ein Griffabschnitt angeordnet sein, so dass mit einer einhändigen Betätigung der Rückzughebel verschoben und die Punktionsnadel somit aus dem Katheter entfernt werden kann.

In einer weiteren vorteilhaft ausgestalteten Erfindung sind gemäß Anspruch 6 Rastmittel vorgesehen. Diese fixieren den Rückzughebel zumindest in seiner nach dem Herausziehen der Punktionsnadel eingenommenen Endposition. Sie können ihn jedoch anfangs auch zusätzlich in seiner im Gebrauchsanfangszustand eingenommenen Position halten. Die Rastmittel, die den Rückzughebel in der erstgenannten Position fixieren, bieten den Vorteil, dass die aus dem Katheter herausgezogene Punktionsnadel nach dem Herausziehen nicht versehentlich wieder mit ihrem spitzen Ende freigegeben werden kann. Dazu wird der Rückzughebel in seiner Endposition fixiert. Durch ein Fixieren des Rückzughebels im Gebrauchsanfangszustand wird erreicht, dass beim Setzen des Katheters der Rückzughebel nicht versehentlich verschoben und die Punktionsnadel somit verfrüht aus dem Katheter herausgezogen werden kann.

Eine weitere vorteilhafte Weiterbildung der Erfindung ist in Anspruch 7 angegeben. Die hier beschriebene Ausgestaltung sorgt dafür, dass die Auszieheinrichtung nicht verfrüht von dem Katheter gelöst werden kann. So ist sichergestellt, dass die Auszieheinrichtung erst nach dem vollständigen Herausziehen der Punktionsnadel aus dem Katheter von letzterem getrennt werden kann. Auch dies verhindert ein frühzeitiges und ungewolltes Herausziehen der Punktionsnadel aus dem Katheter bzw. ein Abziehen der Auszieheinrichtung mit noch in dem Katheter befindlicher Punktionsnadel.

Eine einfache Möglichkeit, die Einzelteile der erfindungsgemäßen Auszieheinrichtung herzustellen, besteht darin, diese aus Kunststoff zu fertigen, vorzugsweise spritzzugießen (Anspruch 8).

Ein gemäß einer in Anspruch 9 angegebenen vorteilhaften Weiterbildung der Erfindung vorgesehenes Griffstück an der Auszieheinrichtung vereinfacht schließlich ein Abziehen der Auszieheinrichtung von dem Katheter nach erfolgtem Herausziehen der Punktionsnadel aus dem Katheter.

Weitere Merkmale und Vorteile der Erfindung ergeben sich aus der nachfolgenden Beschreibung eines Ausführungsbeispiels im Zusammenhang mit den beigefügten Figuren. Dabei zeigen:
- Fig. 1: eine perspektivische Ansicht einer mit einem Katheter verbundenen, erfindungsgemäßen Auszieheinrichtung,
- Fig. 2: eine Schnittdarstellung der Kombination aus Fig. 1, geschnitten entlang einer Längsmittelsenkrechten,

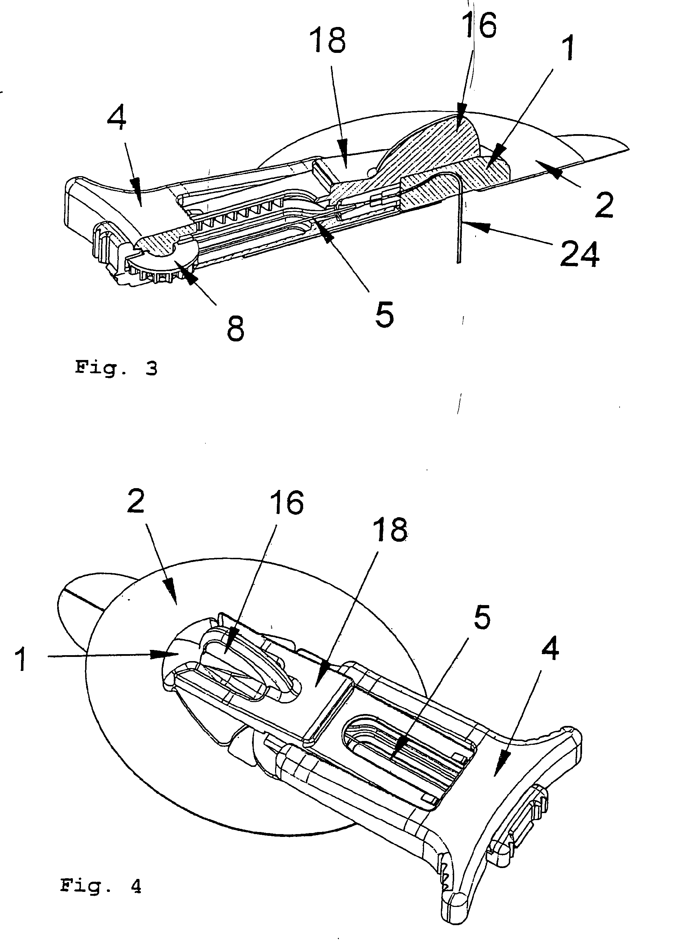

- Fig. 3: eine der Fig. 2 entsprechende Schnittdarstellung mit in eine hintere Position zurückgezogenem Rückzughebel der Auszieheinrichtung,
- Fig. 4: eine perspektivische Darstellung entsprechend der Fig. 1 mit in der hinteren Position befindlichem Rückzughebel der Auszieheinrichtung,

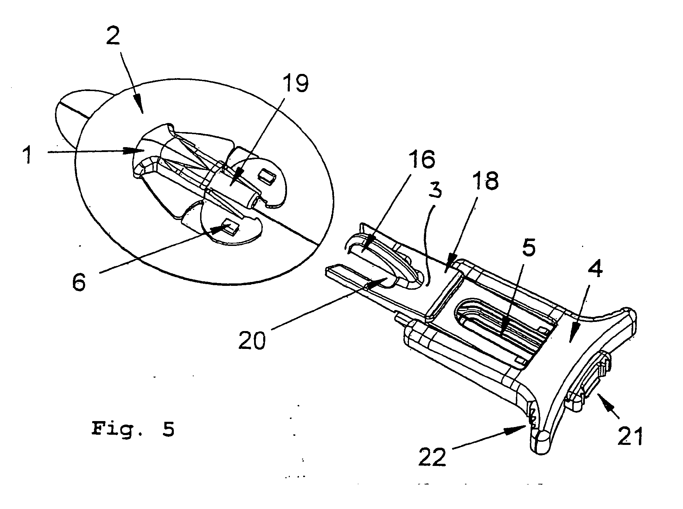

- Fig. 5: in einer Darstellung getrennt voneinander gezeigt einen Katheter sowie die erfindungsgemäße Auszieheinrichtung,
- Fig. 6: in Explosionsdarstellung den Katheter sowie die Auszieheinrichtung in ihren Einzelteilen,
- Fig. 7: eine der Fig. 6 vergleichbare Darstellung, wobei hier die Elemente von unten gezeigt sind.

In den Figuren ist eine erfindungsgemäße Auszieheinrichtung als Nadelzugeinheit 18 bezeichnet. Die Nadelzugeinheit 18 setzt sich im wesentlichen zusammen aus einem lang gestreckten Führungsteil 11 und einem in diesem Führungsteil 11 linear geführten Rückzughebel 4.

Die Nadelzugeinheit 18 ist zum Verbinden mit einem Katheter 1 vorgesehen. Der Katheter 1 ist in diesem Beispiel ein Katheter mit flexiblem Verweilschlauch 24. Der Katheter 1 ist auf einer Klebeplatte 2 montiert, mit welcher er auf der Hautoberfläche eines Patienten fixiert werden kann.

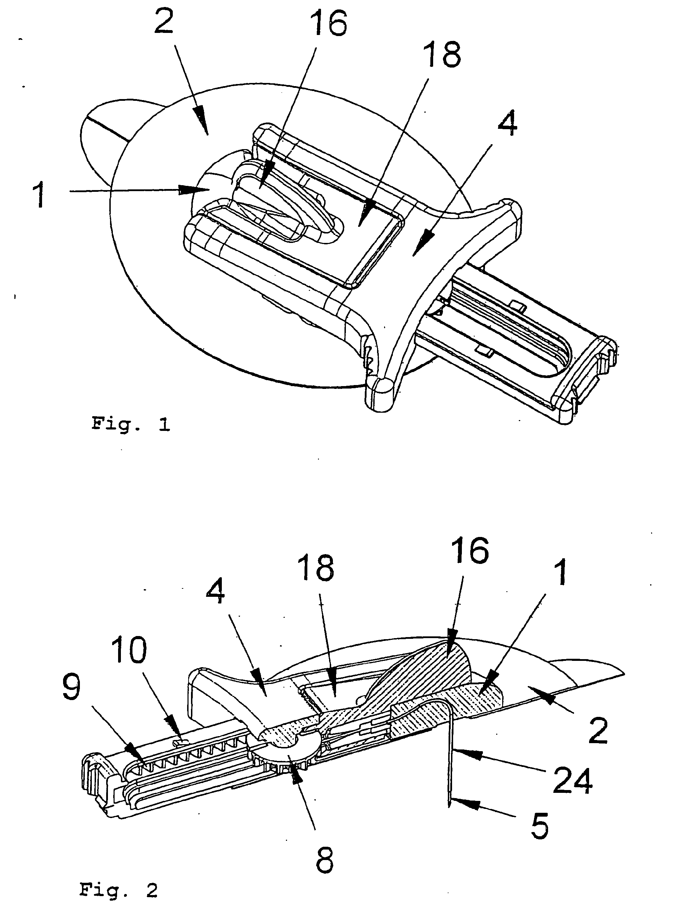

In einer in den Figuren 1 und 2 dargestellten Ausgangsposition in einem Gebrauchsanfangszustand ist die Nadelzugeinheit 18 mit dem Katheter 1 verbunden.

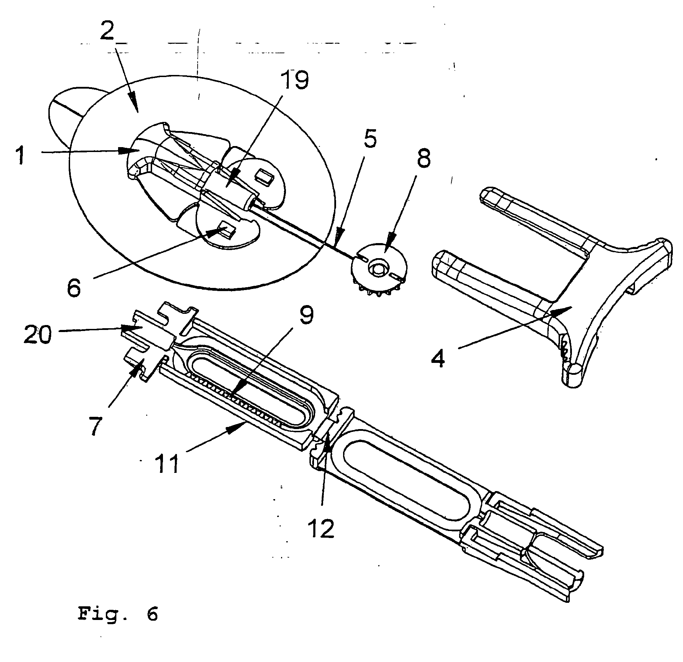

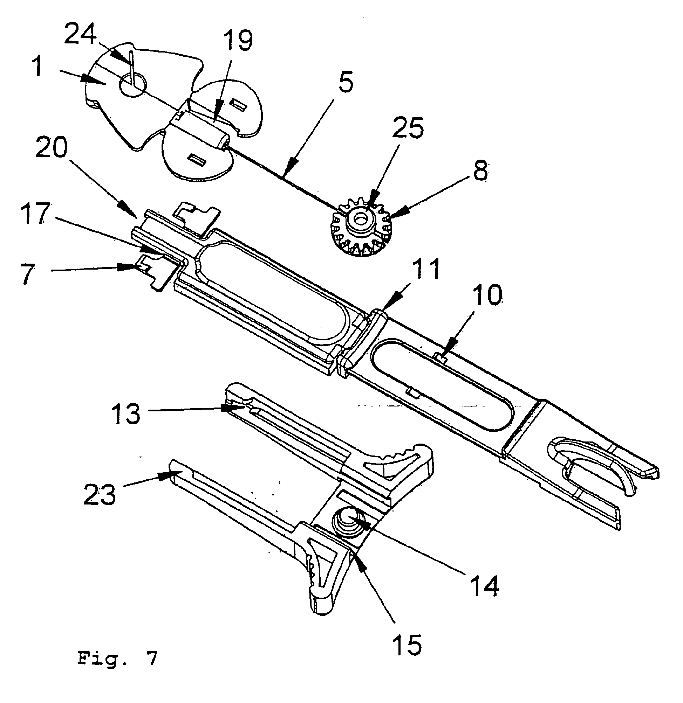

Der genaue Aufbau der Nadelzugeinheit 18 ist besonders gut in den Figuren 6 und 7 zu erkennen. Das Führungsteil 11 ist gleich einem Gehäuse aufgebaut, welches an einer Seitenwand eine lineare Verzahnung in Form einer Zahnstange 9 aufweist. Aus Gründen der einfachen Herstellbarkeit sowie aus Montagegründen ist das Führungsteil 11 aus einem Kunststoffmaterial spritzgegossen und besteht einteilig aus einem Unterteil, in welchem die Zahnstange 9 angeordnet ist sowie einem Deckel, wobei die beiden Elemente, Unterteil und Deckel, über ein Filmscharnier 12 miteinander verbunden sind. In das Führungsteil 11 ist ein Zahnrad 8 so eingesetzt, dass die Umfangsverzahnung des Zahnrades 8 in die Linearverzahnung der Zahnstange 9 eingreift. Das Zahnrad 8 weist einen fest mit diesem verbundenen Aufwickeldorn 25 auf, wobei an dem Aufwickeldorn 25 das rückwärtige Ende einer in den Katheter 1 hineinragenden, den flexiblen Verweilschlauch 24 durchragenden Punktionsnadel 5 festgelegt ist. Das Zahnrad 8 ist bei montierter Nadelzugeinheit 18 auf einem fest an dem Rückzughebel 4 angeordneten Mitnehmerzapfen 14 drehbar gelagert. Der Rückzughebel 4 greift mit Führungsarmen 23 bzw. mit Befestigungshaken 15 in Form von Hinterschnitten an dem Führungsteil 11 an, so dass er in einer geführten Bewegung in dem Führungsteil 11 linear verschoben werden kann. Das Führungsteil 11 weist an einem vorderen Ende einen Verbindungsabschnitt 20 zum Verbinden mit einem komplementär geformten Verbindungsabschnitt 19 an dem Katheter 1 auf. Der Verbindungsabschnitt 19 an dem Katheter 1 ist ein kreiszylinderförmiger Zapfen, der Verbindungsabschnitt 20 an der Nadelzugeinheit 18 ist eine entsprechend komplementär geformte Buchse. Durch den Verbindungsabschnitt 19 des Katheters 1, also durch den kreiszylinderförmigen Zapfen, hindurch erstreckt sich ein Durchgang, durch den hindurch die Punktionsnadel 5 in den flexiblen Verweilschlauch 24 und in diesem bis aus seinem vordersten Ende herausgeführt ist.

An dem Führungsteil 11 sind ferner im vorderen Bereich mit dem Verbindungsabschnitt 20 zwei Haken 7 angeordnet, die über Foliengelenke 17 mit dem Verbindungsabschnitt 20 einstückig verbunden sind.

In dem Deckelteil des Führungsteils 11 sind Stege 10 ausgebildet.

An dem Rückzughebel 4 sind in den vorderen Bereichen der Führungsarme 23 gegenüber der Längserstreckungsrichtung der Führungsarme 23 geneigt verlaufende Führungsbahnen 13 ausgebildet. Der Rückzugshebel 4 verfügt ferner über Griffstücke 22. Ein weiteres Griffstück 21 ist an dem hinteren Ende des Führungsteils 11, also gegenüber dessen Verbindungsabschnitt 20, ausgebildet. Im Bereich des Verbindungsabschnittes 20 weist das Führungsteil 11 einen mit diesem fest verbundenen Griff 16 auf.

An dem Katheter schließlich sind hakenförmige Hintergriffe 6 gebildet.

Die in den Figuren dargestellte, erfindungsgemäße Nadelzugeinheit 18 funktioniert wie folgt:

In einer Ausgangsposition (Fig. 1, Fig. 2) befindet sich der Rückzughebel 4 in einer zum Ende mit dem Verbindungsabschnitt 20 des Führungsteils 11 maximal hin verschobenen Gebrauchsanfangsstellung. Das Zahnrad 8 ist dabei in maximaler Nähe der in dem Verbindungsabschnitt 19 des Katheters 1 befindlichen Öffnung und ist mit dem rückwärtigen Ende der Punktionsnadel 5 fest verbunden. Die flexible Punktionsnadel 5 erstreckt sich durch den Katheter 1 bis über das freie Ende des Verweilschlauches 24 hinaus. Die Nadelzugeinheit 18 ist mit dem Katheter 1 durch das Zusammenwirken der Verbindungsabschnitte 19 des Katheters 1 und 20 der Nadelzugeinheit 18 verbunden, wobei durch Zusammenwirken der Haken 7 an der Nadelzugeinheit 18 und der Hintergriffe 6 an dem Katheter 1 eine Verrastung der Elemente der Nadelzugeinheit 18 und Katheter 1 bewirkt ist.

In diesem Zustand setzt sich der Patient den Katheter 1 bzw. wird der Katheter 1 dem Patienten gesetzt, indem die aus Nadelzugeinheit 18 und Katheter 1 bestehende Einheit an dem Griff 16 der Nadelzugeinheit erfasst und die Punktionsnadel 5 mit dem flexiblen Verweilschlauch 24 in das Gewebe eingedrückt wird. Dann wird der Katheter 1 mit Hilfe der Klebeplatte 2 auf der Haut des Patienten festgelegt. Nun erfolgt das Herausziehen der Punktionsnadel 5 aus dem flexiblen Verweilschlauch 24 und aus dem Katheter 1. Dazu wird der Rückzughebel 4 vorzugsweise an den Griffstücken 22 erfasst, idealerweise mit Zeige- und Mittelfinger einer Hand, und durch Gegendruck, typischerweise mit Hilfe des Daumens, an dem Griffstück 21 des Führungsteils 11 aus seiner vorderen Gebrauchsanfangsposition in eine hintere Endposition gezogen. Beim Überfahren der Stege 10 ergibt sich dabei ein spür- und hörbares Signal, welches dem Anwender zu erkennen gibt, dass die Endposition sicher erreicht ist. Zugleich wird der Rückzughebel 4 dadurch in seiner Endposition verrastet.

Beim Zurückziehen des Rückzughebels 4 wird zugleich das Zahnrad 8 mittels des Mitnehmerzapfens 14 zurückgezogen und durch das Zusammenwirken seiner Umfangsverzahnung mit der Zahnstange 9 zur Rotation gebracht. Im Zuge dieser Rotation wird das hintere Ende der flexiblen Punktionsnadel 5 auf den Aufwickeldorn 25 am Zahnrad 8 aufgewickelt und somit aus dem Katheter 1 bzw. aus dem flexiblen Verweilschlauch 24 gezogen. Ein weiterer Zugeffekt ergibt sich aus der linearen Rückwärtsbewegung des Rückzughebels insgesamt, so dass das Herausziehen der Punktionsnadel 5 aus dem Katheter 1 durch eine Kombination der Rückwärtsbewegung des Rückzughebels 4 und der Drehbewegung des Zahnrades 8 erreicht wird.

Beim Zurückziehen des Rückzughebels 4 in die Endposition passieren die Führungsbahnen 13 letztlich die Haken 7, wobei sie an seitlichen Verlängerungen derselben angreifen. Durch die geneigte Form der Führungsbahnen werden die Haken 7 dann angehoben, wobei sie um die Foliengelenke 17 verschwenken. Hierdurch werden die Haken 7 von den Hintergriffen 6 gelöst, die Nadelzugeinheit kann durch Ziehen an dem Griff 16 vom dem Katheter 1 abgenommen und entsorgt werden.

Das Verhältnis zwischen der Zahnung der Zahnstange 9 und der Umfangsverzahnung des Zahnrades 8 wird dabei so gewählt, dass zum einen die zum Herausziehen der Punktionsnadel 5 aus dem Katheter 1 erforderliche Kraft mit einer normalen Kraftaufbringung auf den Rückzughebel 4 beim Zurückziehen desselben in seine Endposition aufgebracht werden kann, und dass zum anderen die Längserstreckung des Führungsteils 11 möglichst kurz gehalten wird, um die gesamte Baugröße der Nadelzugeinheit 18 so klein wie möglich zu halten.

Die Nadelzugeinheit 18 ist so konzipiert und dimensioniert, dass nach dem Herausziehen der Punktionsnadel 5 aus dem Katheter 1, wenn sich also der Rückzughebel 4 in seiner Endposition befindet, die Spitze der Punktionsnadel 5 im Innern des in dem Führungsteil 11 ausgebildeten Verbindungsabschnittes 20 und damit unterhalb einer Abdeckung 3 befindet. Auf diese Weise ist sichergestellt, dass die Spitze der Punktionsnadel 5 nach dem Herausziehen nicht offen liegt und es nicht zu Verletzungen und damit verbundenen Infektionen kommen kann.

Die Montage der Nadelzugeinheit 18 bzw. der aus Nadelzugeinheit 18 und Katheter 1 bestehenden Einheit erfolgt folgendermaßen:

Zunächst wird an dem aus dem Katheter 1 herausragenden Ende der Punktionsnadel 5 das Zahnrad 8 befestigt, oder es wird in den Katheter 1 eine solche Punktionsnadel 5 eingebracht, an deren Ende bereits ein Zahnrad 8 befestigt ist. Nun wird das Führungsteil 11 in seiner noch geöffneten Position (vgl. Fig. 6 bzw. Fig. 7) mit dem Verbindungsabschnitt 20 über den Verbindungsabschnitt 19 des Katheters 1 gelegt und das Zahnrad 8 wird in die Aufnahme in dem Unterteil des Führungsteils 11 eingelegt, wobei dessen Umfangsverzahnung mit der Verzahnung der Zahnstange 9 in Eingriff gebracht wird. Durch Umbiegen des Deckels entlang des Filmscharniers 12 wird das Führungsteil 11 geschlossen, wobei der Verbindungsabschnitt 20 an dem Verbindungsabschnitt 19 des Katheters 1 fixiert wird. Dabei sorgen nicht dargestellte, geeignete Rastmittel für ein sicheres und festes Verbinden der beiden Teile, Gehäuse und Deckel, des Führungsteils 11. Schließlich wird der Rückzughebel 4 so in das Führungsteil 11 eingesetzt, dass der Mitnehmerzapfen 14 in das Zahnrad 8 eingreift und dass die Führungsarme 23 und die Befestigungshaken 15 mit dem Führungsteil 11 zu einer Führung des Rückzughebels 4 zusammenwirken können. Durch die Befestigungshaken 15 wird der Rückzughebel 4 an dem Führungsteil 11 gehalten.

Nun ist die aus Katheter 1 und Nadelzugeinheit 18 bestehende Einheit in ihrer Gebrauchsanfangsposition gebrauchsfertig und kann vom Patienten eingesetzt werden.

### Bezugszeichenliste

- 1: Katheter
- 2: Klebeplatte
- 3: Abdeckung
- 4: Rückzughebel
- 5: Punktionsnadel
- 6: Hintergriff
- 7: Haken
- 8: Zahnrad
- 9: Zahnstange
- 10: Steg
- 11: Führungsteil
- 12: Filmscharnier
- 13: Führungsbahn
- 14: Mitnehmerzapfen
- 15: Befestigungshaken
- 16: Griff
- 17: Foliengelenk
- 18: Nadelzugeinheit
- 19: Verbindungsabschnitt
- 20: Verbindungsabschnitt
- 21: Griffstück
- 22: Griffstück
- 23: Führungsarm
- 24: Verweilschlauch
- 25: Aufwickeldorn

## Patentansprüche

1. Auszieheinrichtung zum geschützten Herausziehen einer flexiblen Punktionsnadel (5) aus einem Katheter (1), insbesondere einem Katheter (1) mit flexiblem Verweilschlauch (24), nach dem Punktieren, mit einem Verbindungsabschnitt (19) zum Verbinden der Auszieheinrichtung (18) mit dem Katheter (1) in einem Gebrauchsanfangszustand, einem Führungsteil (11) und einem in dem Führungsteil (11) linear bewegbaren Rückzughebel (4), wobei an einem der Elemente Führungsteil (11) oder Rückzughebel (4) eine lineare Verzahnung (9) angebracht ist, die an einer Umfangsverzahnung eines an dem anderen Element - Rückzughebel (4), Führungsteil (11) - drehbar gelagerten Zahnrades (8) angreift, und wobei die Punktionsnadel (5) mit einem aus dem Katheter (1) hervorragenden Ende an dem Zahnrad (8) derart festgelegt ist, dass sie bei einer Rotation des Zahnrades (8) auf einem damit fest verbundenen Aufwickelabschnitt (25) aufgewickelt wird.

2. Auszieheinrichtung nach Anspruch 1, **dadurch gekennzeichnet, dass** die lineare Verzahnung (9) an dem Führungsteil (11) und das Zahnrad (8) an dem Rückzughebel (4) angeordnet sind, wobei das Zahnrad (8) auf einem fest mit dem Rückzughebel (4) verbundenen Zapfen (14) drehbar gelagert ist.

3. Auszieheinrichtung nach einem der Ansprüche 1 oder 2, **dadurch gekennzeichnet, dass** die lineare Verzahnung (9) und die Umfangsverzahnung des Zahnrades (8) so gewählt sind, dass eine vorgewählte Über- bzw. Untersetzung eingestellt ist.

4. Auszieheinrichtung nach einem der vorhergehenden Ansprüche, **dadurch gekennzeichnet, dass** das Führungsteil (11) zumindest in einem Bereich in dem nach einem Herausziehen der Punktionsnadel (5) aus dem Katheter (1) die Spitze der Punktionsnadel (5) liegt, eine Abdeckung (3) zur gedeckten Aufnahme der Spitze der Punktionsnadel (5) aufweist.

5. Auszieheinrichtung nach einem der vorhergehenden Ansprüche, **dadurch gekennzeichnet, dass** der Rückzughebel (4) einen Griffabschnitt (21) zum Angreifen beim Verschieben (Zurückziehen) des Rückzughebels (4) aufweist.

6. Auszieheinrichtung nach einem der vorhergehenden Ansprüche, **dadurch gekennzeichnet, dass** Rastmittel (10) vorgesehen sind, die den Rückzughebel (4) relativ zu dem Führungsteil (11) wenigstens in seiner nach dem Herausziehen der Punktionsnadel (5) eingenommenen Endposition fixieren.

7. Auszieheinrichtung nach einem der vorhergehenden Ansprüche, **dadurch gekennzeichnet, dass** sie Rastmittel (6, 7) zum Verrasten des Verbindungsabschnittes (19) mit einem passenden Gegenabschnitt (20) des Katheters (1) aufweist sowie Mittel (13) zum Lösen der Rastmittel (6, 7) zum Entfernen der Auszieheinrichtung (18) von dem Katheter (1), wobei die Mittel (13) zum Lösen der Rastmittel (6, 7) derart mit dem Rückzughebel (4) in Verbindung stehen, dass sie die Rastmittel (6, 7) erst bei in einer maximal von dem Katheter (1) entfernten Endposition befindlichem Rückzughebel (4) lösen.

8. Auszieheinrichtung nach einem der vorhergehenden Ansprüche, **dadurch gekennzeichnet, dass** zumindest der Rückzughebel (4) und das Führungsteil (11) aus Kunststoff bestehen, vorzugsweise aus diesem Material spritzgegossen sind.

9. Auszieheinrichtung nach einem der vorhergehenden Ansprüche, **dadurch gekennzeichnet, dass** sie ein Griffstück (16) zum Abziehen der Auszieheinrchtung (18) von dem Katheter (1) aufweist.

## Claims

1. Extraction device for the protected extraction of a flexible puncture needle (5) from a catheter (1), particularly a catheter (1) having a flexible self-retaining tube (24), following puncturing, having a connecting section (19) for the connection of the extraction device (18) to the catheter (1) in an initial use state, a guide part (11) and a retraction lever (4) linearly movable in the guide part (11), a linear gearing (9) being fitted to one of the elements guide part (11) or retraction lever (4) and engaging on a cylindrical gearing of a gear wheel (8) mounted in rotary manner on the other element retraction lever (4) or guide part (11), the puncture needle (5) being fixed by an end projecting from the catheter (1) to the gear wheel (8) in such a way that during a reduction of said gear wheel (8) it is wound onto a wind-on section (25) rigidly connected thereto.

2. Extraction device according to claim 1, **characterized in that** the linear gearing (9) is located on the guide part (11) and the gear wheel (8) on the retraction lever (4), the gear wheel (8) being mounted in rotary manner on a lug (14) rigidly connected to the retraction lever (4).

3. Extraction device according to one of the claims 1 or 2, **characterized in that** the linear gearing (9) and cylindrical gearing of the gear wheel (8) are selected in such a way that there is a preselected speed increase or decrease.

4. Extraction device according to one of the preceding claims, **characterized in that** the guide part (11), at least in an area where the tip of the puncture needle (5) is located following the extraction of said puncture needle (5) from the catheter (1), has a cover (3) for the concealed reception of the tip of puncture needle (5).

5. Extraction device according to one of the preceding claims, **characterized in that** the retraction lever (4) has a grip section (21) for gripping during the displacement (retraction) of the retraction lever (4).

6. Extraction device according to one of the preceding claims, **characterized in that** locking means (10) are provided, which fix the retraction lever (4) relative to the guide part (11) at least in its end position assumed following the extraction of puncture needle (5).

7. Extraction device according to one of the preceding claims, **characterized in that** it has locking means (6, 7) for locking the connecting section (19) to a matching countersection (20) of catheter (1), as well as means (13) for releasing the locking means (6, 7) for removing extraction device (18) from catheter (1), the means (13) for releasing the locking means (6, 7) being connected to the retraction lever (4) in such a way that they only release the locking means (6, 7) when the retraction lever (4) is in an end position at the maximum distance from catheter (1).

8. Extraction device according to one of the preceding claims, **characterized in that** at least the retraction lever (4) and guide part (11) are made from plastic and are preferably injection moulded from said material.

9. Extraction device according to one of the preceding claims, **characterized in that** it has a grip (16) for removing extraction device (18) from catheter (1).

## Revendications

1. Dispositif d'extraction pour extraire de façon protégée une aiguille à ponction (5) flexible d'un cathéter (1), en particulier un cathéter (1) avec tuyau d'arrêt (24) flexible, après la ponction, avec un tronçon de liaison (19) pour la liaison du dispositif d'extraction (18) avec le cathéter (1) dans un état initial d'utilisation, une partie de guidage (11) et un levier de recul (4) linéairement mobile dans la partie de guidage (11), une denture (9) linéaire étant placée sur l'un des éléments partie de guidage (11) ou levier de recul (4), laquelle denture s'engage sur une denture périphérique d'une roue dentée (8) logée de façon rotative sur l'autre élément - levier de recul (4), partie de guidage (11) -, et l'aiguille de ponction (5) étant fixée par une extrémité dépassant du cathéter (1) sur la roue dentée (8) de telle sorte qu'elle est enroulée dans le cas d'une rotation de la roue dentée (8) sur un tronçon d'enroulement (25) relié de façon de fixe à celle-ci.

2. Dispositif d'extraction selon la revendication 1, **caractérisé en ce que** la denture (9) linéaire et la roue dentée (8) sont disposées respectivement sur la partie de guidage (11) et le levier de recul (4), la roue dentée (8) étant logée de façon rotative sur un pivot (14) relié de façon fixe au levier de recul (4).

3. Dispositif d'extraction selon l'une quelconque des revendications 1 ou 2, **caractérisé en ce que** la denture (9) linéaire et la denture périphérique de la roue dentée (8) sont choisies de telle sorte qu'une multiplication ou une démultiplication présélectionnée est réglée.

4. Dispositif d'extraction selon l'une quelconque des revendications précédentes, **caractérisé en ce que** la partie de guidage (11) présente un revêtement (3) pour le logement couvert de l'extrémité de la pointe de l'aiguille de ponction (5) au moins dans une zone dans laquelle se trouve la pointe de l'aiguille de ponction (5) après une extraction de l'aiguille de ponction (5) du cathéter (1).

5. Dispositif d'extraction selon l'une quelconque des revendications précédentes, **caractérisé en ce que** le levier de recul (4) présente une partie poignée (21) pour la saisie lors du déplacement (recul) du levier de recul (4).

6. Dispositif d'extraction selon l'une quelconque des revendications précédentes, **caractérisé en ce que** des moyens d'encliquetage (10) sont prévus, lesquels fixent le levier de recul (4) par rapport à la partie de guidage (11) au moins dans sa position finale occupée après l'extraction de l'aiguille de ponction (5).

7. Dispositif d'extraction selon l'une quelconque des revendications précédentes, **caractérisé en ce qu'**il présente des moyens d'encliquetage (6, 7) pour l'encliquetage du tronçon de liaison (19) avec une contrepartie (20) adaptée du cathéter (1) et des moyens (13) pour le détachement des moyens d'encliquetage (6, 7) destinés à l'enlèvement du dispositif d'extraction (18) du cathéter (1), les moyens (13) pour détacher les moyens d'encliquetage (6, 7) étant en liaison avec le levier de recul (4) de telle sorte qu'ils détachent les moyens d'encliquetage (6, 7) seulement lorsque le levier de recul (4) se trouve dans une position finale éloignée au maximum du cathéter (1).

8. Dispositif d'extraction selon l'une quelconque des revendications précédentes, **caractérisé en ce que** au moins le levier de recul (4) et la partie de guidage (11) sont en plastique, de préférence moulés par injection à partir de ce matériau.

9. Dispositif d'extraction selon l'une quelconque des revendications précédentes, **caractérisé en ce qu'**il présente une poignée (16) pour l'enlèvement du dispositif d'extraction (18) du cathéter (1).
